# EUROPEAN PATENT APPLICATION

(11) **EP 4 556 572 A1**
(43) Date of publication of application: **21.05.2025**
(21) Application number: 23210819.1
(22) Date of filing: 20.11.2023
(51) Int. Cl.: C12Q 1/6806

(54) **METHOD FOR PURIFYING ONE OR MORE TARGET NUCLEIC ACID(S)**

(71) Applicant: Sartorius BIA Separations d.o.o., 5270 Ajdovscina (SI)
(72) Inventor: Miklavcic, Rok, 5261 Sempas (SI); Cernigoj, Urh, 5270 Ajdovscina (SI)
(74) Representative: Müller-Boré & Partner Patentanwälte PartG mbB

(57) **Abstract**

The present invention relates to a method for purifying one or more target nucleic acid(s), comprising the steps of providing a solution containing the target nucleic acid(s), providing an anion exchange solid phase, characterized in that a switch between an overall positive charge of said anion exchange solid phase to a neutral charge or a negative charge occurs in a pH range of between pH 3 and pH 9, contacting the solution containing the target nucleic acid(s) to said anion exchange solid phase under conditions that allow binding of said target nucleic acid(s) to said solid phase, and eluting the nucleic acid(s) from the anion exchange solid phase using an elution buffer having a pH that is close to or higher than the pH at which the switch between an overall positive charge of said anion exchange solid phase to a neutral charge or a negative charge occurs.

## Description

The present invention relates to a method for purifying one or more target nucleic acid(s), comprising the steps of providing a solution containing the target nucleic acid(s), providing an anion exchange solid phase, characterized in that a switch between an overall positive charge of said anion exchange solid phase to a neutral charge or a negative charge occurs in a pH range of between pH 3 and pH 9, contacting the solution containing the target nucleic acid(s) to said anion exchange solid phase under conditions that allow binding of said target nucleic acid(s) to said solid phase, and eluting the nucleic acid(s) from the anion exchange solid phase using an elution buffer having a pH that is close to or higher than the pH at which the switch between an overall positive charge of said anion exchange solid phase to a neutral charge or a negative charge occurs.

Analysis and diagnostics (e.g. electrophoretic analysis, mass spectrometry analysis, sequencing, PCR reaction, or the like), formulation, and therapeutic use/application of nucleic acids (e.g. electroporation, transfection, or the like) typically require purified nucleic acid samples (e.g. plasmid DNA, small interfering RNA, messenger RNA) having a low salt content. However, a low salt content is usually not directly achieved during purification itself. Typical examples are anion exchange chromatography and hydrophobic interaction chromatography, where nucleic acid solutions with a high salt concentration (e.g. > 0.15 M NaCl, > 0.1 M (NH₄)₂SO₄) are produced. To lower salt concentration and concentrate nucleic acids, additional desalting and concentrating steps are applied after the purification step. Additionally, desalting is applied in between industrial purification steps to enable sample transfer between different purification methods. For its industrial use, a method for purification/desalting and concentration should therefore preferably be easily scalable.

In this context, methods currently employed to purify/desalt (and/or concentrate) nucleic acids include filtration methods (e.g. tangential flow filtration, ultrafiltration); dialysis; precipitation (using ethanol or isopropanol in combination with salts or using LiCl for RNA precipitation); size-exclusion (gel filtration) chromatography (e.g., agarose-based column); solid phase extraction (e.g. silica columns); and anion exchange chromatography.

The applicability of these current methods for nucleic acid purification/desalting and their advantages/disadvantages are discussed below.

Dialysis enables efficient, low-shear force desalting/buffer exchange, but the process is very slow, requires huge amounts of buffers compared to sample volume and is not easily scalable. Furthermore, dialysis does not allow straightforward concentration of the sample.

A faster process for desalting is based on size exclusion principles, for example using Sephadex^{™} G-25 medium. A size exclusion approach however does not enable sample concentration and can furthermore even lead to sample dilution due to diffusion of the sample in the medium. Sample application volume is also an issue with size exclusion chromatography (SEC), a typical sample application volume being less than 1/10 of desalting medium (column) volume. Pore size and desalting medium need to be tailored/selected properly for molecules of different size. Another limitation of size exclusion with packed columns is low flowrate due to high back-pressure, making the process longer compared to chromatography with solid phases with convection-based mass transfer.

Ultrafiltration (for example Amicon^{®} Ultra centrifugal filters) enables concentration of target molecules, but they are susceptible to relatively high shear forces and sometimes decreased recoveries due to non-specific adsorption to ultrafiltration membranes (materials) or even due to a partial permeability of the membranes (materials) for some of the target molecules.

Tangential flow filtration (TFF) is accepted in the art as a method which is often used in between purification steps or as the last step in nucleic acid downstream processing for desalting, buffer exchange and concentration of the target molecules. The process is scalable but is prone to relatively high shear forces due to the specific design of the system. This limitation can especially be crucial when very large biomolecules, susceptible to shearing (e.g. plasmid DNA, mRNA), are processed. In addition, operational time for TFF and need for excess amounts of target buffer are sometimes a drawback. Another important drawback of TFF is that it cannot be used for desalting nano- and microgram quantities of nucleic acids and it does not enable desalting of a very large span of molecule sizes using the same membrane/material, as the material, pore size, and composition/chemistry needs to be tailored for each individual molecule/group of molecules.

Filtration methods and dialysis all rely on large dilutions of salts and buffer components to perform desalting/buffer exchange. However, such methods cannot achieve complete elimination of unwanted trace salts/components from biomolecule preparations. Additionally, the materials used need to be properly selected for each individual sample, for example by choosing the right membrane material (PES, cellulose, or the like) with appropriate pore size to prevent loss of target molecule.

Only precipitation (as used e.g. in QIAgen kits, for example QIAprecipitator Module) and solid phase extraction (e.g. using silica columns) enables complete elimination of unwanted salts from nucleic acid preparations. However, both methods also have considerable disadvantages. Precipitation is not easily scalable and requires an additional step for resolubilization of the target molecule. In addition, organic solvents in combination with salts are usually needed to induce precipitation.

Silica columns (for example QIAgen silica columns or Thermo Scientific silica columns) act as solid phase extraction media. Nucleic acids are bound to the solid phase in a buffer containing for example high guanidine concentration, then they are washed with organic solvents and eluted with water. However, the use of organic solvent is needed, which is an obvious drawback of the method.

Standard anion exchangers are used for desalting of nucleic acids, where the elution is performed at alkaline conditions, for example using ammonia. However high pH (e.g. pH > 10) is required to achieve elution from these solid phases which is not desired for nucleic acids as it promotes their degradation. Despite simple process design, such treatment cannot be extended to elution conditions at neutral pH, where nucleic acids are considerably more stable, due to the nature of standard anion exchangers.

Accordingly, all of the above methods entail one or the other drawbacks, as outlined above.

Therefore, the technical problem underlying the present invention is the provision of an easy method for the purification/desalting or simultaneous purification/desalting and concentration of a target nucleic acid, enabling a scaleup from nanograms up to kilograms of nucleic acid and offering the use of different nucleic acid modalities from few kDa to several MDa of size, wherein said method can be performed at mild conditions with respect to pH, temperature, and shear forces, does not require the use of organic solvents, and displays a high nucleic acid recovery rate.

The solution to the above technical problem is achieved by the embodiments characterized in the claims.

In particular, the present invention relates to a method for purifying one or more target nucleic acid(s), comprising the steps of:
(a) providing a solution containing the target nucleic acid(s);
(b) providing an anion exchange solid phase, characterized in that a switch between an overall positive charge of said anion exchange solid phase to a neutral charge or a negative charge occurs in a pH range of between pH 3 and pH 9;
(c) contacting the solution containing the target nucleic acid(s) to said anion exchange solid phase under conditions that allow binding of said target nucleic acid(s) to said solid phase; and
(d) eluting the nucleic acid(s) from the anion exchange solid phase using an elution buffer having a pH that is close to or higher than the pH at which the switch between an overall positive charge of said anion exchange solid phase to a neutral charge or a negative charge occurs.

Nucleic acids that can function as the target nucleic acid in the method of the present invention are not particularly limited and include any types and forms of nucleic acid that might be of interest. This includes in particular any single-stranded or double-stranded nucleic acids, any nucleic acids in linear or circular form, and any types and forms of DNA, RNA, or DNA/RNA-hybrids. In specific embodiments, the nucleic acid is selected from the group consisting of genomic DNA, cDNA, chromosomal DNA, plasmid DNA, DNA oligonucleotides, messenger RNA (mRNA), transfer RNA (tRNA), ribosomal RNA (rRNA), small interfering RNA (siRNA), circular RNA, self-amplifying RNA, RNA oligonucleotides, and mixtures thereof.

In specific embodiments, the nucleic acid(s) used can be artificially-produced using naturally-occurring and/or modified nucleobases, nucleotides, or the like. The size of nucleic acids that can function as the target nucleic acid in the method of the present invention is not particularly limited and could range from 2 kDa up to 500 MDa. Further, the solution provided in step (a) of the method of the present invention can contain the nucleic acid in any amount. This includes low concentration starting material, e.g., solutions that contain the nucleic acid in an amount of only 100 µg/mL or lower.

In specific embodiments, purifying the one or more target nucleic acid(s) according to the present invention comprises the removal of
(i) ionic salts, and/or
(ii) undesired organic compounds, and/or
(iii) undesired organic solvents, and/or
(iv) biomolecules other than the target nucleic acid(s),
that may be present in the solution provided in step (a).

Accordingly, the solution provided in step (a) of the method of the present invention can contain
(i) one or more ionic salt(s), and/or
(ii) one or more undesired organic compound(s), and/or
(iii) one or more undesired organic solvent(s), and/or
(iv) one or more biomolecule(s) other than the target nucleic acid.

Thus, the method of the present invention can be a method of removing or decreasing one or more ionic salt(s) from a solution containing the target nucleic acid(s), i.e., said method can be a method of desalting the target nucleic acid(s). However, the additional or alternative removal or decrease of one or more of one or more undesired organic compound(s), and/or one or more undesired organic solvent(s), and/or one or more biomolecule(s) other than the target nucleic acid is also encompassed herein and can be part of the method of the present invention.

Respective ionic salt(s) are not particularly limited and include any ionic salts that might be present in a given nucleic acid solution. In specific embodiments, the ionic salt(s) is/are selected from the group consisting of organic or inorganic salts containing one or more ion(s), selected from the group consisting of Na⁺, K⁺, Cl⁻, Li⁺, acetate, phosphate(V), Mg²⁺, Ca²⁺, Mn²⁺, pyrophosphate, ammonium, sulphate(VI), EDTA, citrate, thiocyanate, nitrate (V), and guanidinium. Specific examples of respective salts include ionic salts selected from the group consisting of NaCl, KCI, Na-acetate, Na-phosphate, MgCl₂, CaCl₂, Na-pyrophosphate, K-pyrophosphate, ammonium sulphate, EDTA, guanidinium hydrochloride, and guanidinium thiocyanate.

These ionic salt(s), the quantity of which in the nucleic acid(s) solution is to be removed or decreased, can be contained in the solution provided in step (a) of the method of the present invention in any given amount. In specific embodiments, the salt(s) are contained in said solution in a concentration of 0.001 M or higher, 0.01 M or higher, or 0.1 M or higher.

Undesired organic compound(s) and/or organic solvent(s) can be contained in the solution provided in step (a) of the method of the present invention e.g. in an amount of 0.001% (v/v) or more for each organic compound or organic solvent. These undesired organic compounds or organic solvents can be separated from the nucleic acid(s) in the method of the present invention. Exemplary organic compounds/solvents in this respect can be selected from the group consisting of acetonitrile, formamide, formaldehyde, ethanol, 1-propanol, 2-propanol, urea, surfactants, (e.g. poloxamer, tween, triton), sorbitol, saccharose, spermine, and spermidine. Of note, organic solvents can typically derive from preceding reverse phase chromatography procedures.

Further, as indicated above, the solution provided in step (a) of the method of the present invention can further contain one or more biomolecule(s) other than the target nucleic acid which can be separated from the nucleic acid in the method of the present invention. Exemplary biomolecules in this respect can be selected from the group consisting of nucleotides (e.g. nucleotide monophosphates, nucleotide diphosphates, or nucleotide triphosphates), proteins, enzymes (e.g. DNase, RNase, pyrophosphatase, RNA-polymerase), lipids, carbohydrates, and other non-target nucleic acids (e.g. oligonucleotides, template DNA, rRNA, small RNA).

In step (a) of the method of the present invention, a solution containing the target nucleic acid(s) is provided. In specific embodiments, this solution has a conductivity of between 1 and 300 mSi/cm, preferably between 2 and 150 mSi/cm, more preferably between 5 and 100 mSi/cm. Of note, all specific conductivity values taught herein are conductivity values as measured at 25°C. Further, in preferred embodiments, the solution provided in step (a) of the method of the present invention has a pH that is the same or lower as the pH at which the switch between an overall positive charge of the anion exchange solid phase to a neutral charge or a negative charge occurs, e.g. a pH between pH 3 and pH 9, preferably between pH 4 and pH 7.5, more preferably between pH 5 and pH 7, provided that the condition of the pH being the same or lower as the pH at which the switch between an overall positive charge of the anion exchange solid phase to a neutral charge or a negative charge occurs is met.

In this context, as used herein, the term "lower pH" as compared to a reference pH refers to a pH that is at least 0.5 to 2 pH units lower than the reference pH, preferably at least 0.5 to 4 pH units lower than the reference pH. Likewise, as used herein, the term "higher pH" as compared to a reference pH refers to a pH that is at least 0.5 to 2 pH units higher than the reference pH, preferably at least 0.5 to 4 pH units higher than the reference pH. Further, the term "a pH close to" a reference pH refers to a pH that is up to 1 pH unit lower or higher than the reference pH, preferably up to 0.5 pH units lower or higher than the reference pH, more preferably up to 0.3 pH units lower or higher than the reference pH, more preferably up to 0.2 pH units lower or higher than the reference pH, more preferably up to 0.1 pH units lower or higher than the reference pH. Further, the term "a pH close to" a reference pH can also refer to a pH that is the same as the reference pH. These definitions, as provided in the context of pH values, equally apply to pKa and pl values defined herein.

The anion exchange solid phase used in the method of the present invention, provided in step (b) of said method, must be positively charged at a specific pH, wherein a switch between an overall positive charge to a neutral charge or negative charge occurs in a pH range between pH 3 and pH 9, preferably between pH 4 and pH 7.5, more preferably between pH 5 and pH 7. The switch occurs with special ligands, bound on the surface of the anion exchange solid phase.

These ligands contain one or more specific functional groups. The pKa values of at least one of these specific functional groups (being in a state of conjugated acids) on the ligand is between pKa 3 and pKa 9, preferably between pKa 4 and pKa 7.5, more preferably between pKa 5 and pKa 7. In the case of conjugated acids, a ligand is positively charged at pH values below the pKa ("binding mode" for nucleic acids) and neutral or negatively charged at pH values above its pKa ("elution mode"). The positive charge of the solid phase interacts with the backbone of nucleic acids, specifically with the negatively charged phosphate backbone. Elution of bound nucleic acids is achieved at pH values close to or higher than the pKa value of the ligand. Accordingly, elution at neutral pH is possible.

The ligands can be selected from the group consisting of substituted aliphatic amines (e.g., be glucosamine (pKa = 8.2) or tricine (pKa = 8.15)), and nitrogen-containing heterocycles (e.g. aromatic nitrogen-containing heterocycles). In the case of nitrogen-containing heterocycles, the pKa values of specific functional group on the ligand depend on the number and position of nitrogen atoms inside the ring. Preferably, the nitrogen-containing heterocycle is selected from the group consisting of purines, pyrimidines, imidazoles, pyridines, diazoles, triazoles, morpholines, and tetrazoles, wherein imidazole, imidazole derivatives, pyridine, and pyridine derivatives are particularly preferred. More preferably, the nitrogen-containing heterocycle is selected from the group consisting of histamine and mercaptopyridine, e.g. 4-mercaptopyridine. According to the present invention, the ring structure is such that the pKa is between pKa 3 and pKa 9, preferably between pKa 4 and pKa 7.5, more preferably between pKa 5 and pKa 7. As used herein, the term "nitrogen-containing heterocycle ligand having a pKa value of 9 or less" means that the at least one nitrogen-containing heterocycle has at least one nitrogen atom with a pKa value of the conjugate acid of pKa 9 or less.

In some situations, the isoelectric point (pl) is used to describe the charge distribution of the ion-exchanging solid phase, especially when more than one different acid/base functional groups are present on the same material. The pl value is herein defined as the pH at which a material carries no net electrical charge or is electrically neutral in the statistical mean. The pl value of the anion exchange solid phase and/or the ligand coupled to said solid phase is preferably a pl of 3 or more and 9 or less, preferably a pl of 4 or more and 7.5 or less, more preferably a pl of 5 or more and 7 or less.

In specific embodiments, the anion exchange solid phase provided in step (b) of the method of the present invention is a chromatographic solid phase, e.g. a solid phase that is selected from the group consisting of chromatographic monoliths, membranes, beads (porous and non-porous) and fibers. Alternatively, the solid phase is a chromatographic column comprising a solid stationary phase.

The method of the present invention can employ a solid phase comprising a specific anion-exchanging ligand as described above. In a preferred embodiment, the solid phase is a support matrix having the ligands bound thereto. The type of solid phase is not particularly limited as long as it is suitable to be used for liquid chromatography and can comprise the ligands.

Preferably, the solid phase has on its surface chemical functional groups, to which the ligands can bind or be bound. These groups can already be present on the surface of the solid phase or can be introduced in a suitable manner. According to the invention, therefore, one can either use a chromatographic solid phase that originally comprises functional groups (such as glycidyl-containing polymethacrylates), or a chromatographic support into which suitable functional groups can be introduced by means of a surface modification known to the person skilled in the art. In this connection, examples of known surface modifications include substitution and addition reactions, reaction with functional epoxides, activated acids or active esters, activation by means of plasma treatment, e-beam (electron beam) treatment, gamma irradiation, coating, hydrolysis, aminolysis, oxidation, reduction, reaction with functional carbenes and/or nitrenes, etc.

According to an embodiment of the present invention, the solid phase comprises at least one material selected from the group composed of natural or synthetic fibers, (polymer) membranes, natural or synthetic non-porous particles, natural or synthetic perfusion-based particles, natural or synthetic monolithic solid supports, polymer gels, films, nonwovens and wovens. The solid phase could be from organic or inorganic material. In a preferred embodiment, the solid phase is an organic-based monolithic solid support or membrane.

Examples of natural or synthetic fibers that can be used as a material for the solid phase include electrospun cellulose fibers (such as "Fibre" from Cytiva or "AstreAdept" technology from Astrea Bioseparations), polyester fibers (such as "Winged Fibers" from the firm Allasso Industries, composed of polyethylene terephthalate (PET) or "4DG^{™} Fibers" from the firm Fiber Innovation Technology, composed of polyethylene terephthalate) and fibers comprising cellulose derivatives, Nylon, polyethylene (PE), polyamide (PA), sulfone (PES), polyvinylidene difluoride (PVDF), polytetrafluoroethylene (PTFE), polypropylene (PP), polysulfone, and polycaprolactone as a structuring component, wherein the materials can be used individually or in corresponding combinations.

Examples of (polymer) membranes that can be used as a material for the solid phase include membranes comprising cellulose, cellulose derivatives, Nylon, polyester, polyethylene (PE), polyamide (PA), sulfone (e.g., polyethersulfone (PES)), polyvinylidene difluoride (PVDF), polytetrafluoroethylene (PTFE), polypropylene (PP) and polysulfone as a structuring component, wherein the materials can be used individually or in corresponding combinations. Preferably, membranes based on cellulose and cellulose derivatives, in particular cellulose hydrate membranes, or polyethylene membranes are used.

Examples of polymer gels that can be used as a material for the solid phase include agarose, dextran, cellulose, polymethacrylates, polyvinyl ethers, polyesters, polyacrylamides, polystyrene-divinylbenzene copolymers, silica dextran, agarose acrylamides and dextran acrylamides.

Examples of films and wovens that can be used as a material for the solid phase include films and wovens composed of the above-mentioned polymer materials that can be used for the (polymer) membranes. Examples of nonwovens that can be used as a material for the solid phase include polyester/polypropylene/polyamide nonwovens (such as "Pluratexx 2317 S" from the firm Freudenberg) and the above polymer materials that can be used for the (polymer) membranes.

Examples of monolithic solid supports include molded monoliths, synthesized, for example, from a single polymer mixture composed of poly(glycidyl methacrylate -co- ethylene dimethacrylate) or from a single polymer mixture composed of polystyrene-divinylbenzene. A particular example are CIM (Convective Interaction Media) monolithic chromatographic columns from Sartorius BIA Separations d.o.o. Another example are so-called hydrogels, which represent monoliths synthesized first as a macro-skeleton with a secondary ligand-bearing polymer phase synthesized on top of it. Solid support materials may be provided in a housing to facilitate performance of chromatography.

The above-described materials may intrinsically comprise an anion-exchanging ligand or may be functionalized with an anion-exchanging ligand. Binding of a ligand may be achieved via non-covalent or covalent bonding; preferably covalent bonding is used. According to the present invention, specific ligands are bound to the surface of the material for the solid phase either directly or via polymeric spacer elements so as to form the solid phase.

In the solid phase used in the method of the present invention, the ligands are preferably bound via polymeric spacer elements between the surface of the material for the solid phase and the multimodal ligand to form the solid phase. The binding between the surface of the material for the solid phase and the spacer elements preferably takes place or has taken place via functional groups originally present or produced by surface modification of the solid phase. By means of the polymeric spacer elements, which may serve as binding units between the surface of the material for the solid phase and the ligands in the solid phase, it is advantageously possible to obtain high ligand densities, which allow high binding capacity for the target substance.

pKa is the acid dissociation constant (also known as acidity constant, or acidionization constant) and is a quantitative measure of the strength of an acid in solution. pKa within the scope of the present application is defined as a pH value at standard conditions, where half of the functional groups, being part of the ligand, are positively charged and half of it is not charged. The pKa value of the ligand can, for example, be determined by potentiometric titration at room temperature.

In step (c) of the method of the present invention, the solution containing the target nucleic acid(s) is contacted to the anion exchange solid phase provided in step (b) under conditions that allow binding of said target nucleic acid(s) to said solid phase. By way of this contacting step, the nucleic acid(s) contained in said solution bind(s) to said solid phase. This can be achieved under various conditions, which depend on the solid phase and/or ligand used, as well as the type/size of target nucleic acid(s). The conditions of contacting, specifically the pH and conductivity of the solution from step (a), must enable the binding of target nucleic acid(s) to the solid phase.

Thus, in cases where suitable conditions of contacting the solution containing the target nucleic acid(s) to the anion exchange solid phase provided in step (b) that allow binding of said target nucleic acid(s) to said solid phase are not yet met, the method of the present invention can further comprise prior to step (c) a step of adjusting the solution provided in step (a) to a pH and conductivity that allow binding of said target nucleic acid(s) to said solid phase. Suitable conductivities and pH values are as defined above for this solution. In particular, the solution provided in step (a) can be adjusted to a conductivity of between 1 and 300 mSi/cm, preferably between 2 and 150 mSi/cm, more preferably between 5 and 100 mSi/cm, and/or to a pH that is the same or lower as the pH at which the switch between an overall positive charge of the anion exchange solid phase to a neutral charge or a negative charge occurs, e.g. a pH between pH 3 and pH 9, preferably between pH 4 and pH 7.5, more preferably between pH 5 and pH 7, provided that the condition of the pH being the same or lower as the pH at which the switch between an overall positive charge of the anion exchange solid phase to a neutral charge or a negative charge occurs is met. In relates embodiments, the solution provided in step (a) can be adjusted to a conductivity of at least 10 mSi/cm, at least 20 mSi/cm, or at least 50 mSi/cm, with suitable upper boundaries in combination with these values being 300 mSi/cm, 150 mSi/cm, or 100 mSi/cm. These embodiments can be of particular relevance in cases there purification according to the present invention encompasses the removal of undesired organic compounds, and/or undesired organic solvents, and/or biomolecules other than the target nucleic acid(s), and wherein the solution provided in step (a) of the method of the present invention has a low conductivity, e.g. a conductivity of 20 mSi/cm or lower, 10 mSi/cm or lower, 5 mSi/cm or lower, or 2 mSi/cm or lower.

Binding of target nucleic acids onto the anion exchange solid phase is dependent on the pH, conductivity, and type of anion-exchange solid phase. Binding of nucleic acids to anion exchange solid phase is usually achieved at pH values below the pKa of the ligand of the solid phase and it becomes stronger with decreasing conductivity. Thus, the above adjustment step can be omitted in cases where the solution provided in step (a) already has a pH that is the same or lower than the pH at which the switch between an overall positive charge of the anion exchange solid phase to a neutral charge or a negative charge occurs.

In cases where the adjustment of the solution provided in step (a) is needed, this can be done with the dilution of the provided solution from step (a) using water or a dilution buffer. The goal of this step is to decrease the conductivity and/or change a pH as outlined above. The dilution factor is preferably 1:1000, more preferably 1:100, more preferably 1:10, most preferably 1:1. Suitable buffers are not particularly limited and are known in the art, including acetate, citrate, MES, HEPES, TRIS, bis-tris propane, phosphate, or the like. The concentration of dilution buffer is between 1 and 500 mM, preferably 2 to 200 mM, more preferably between 5 and 100 mM. The pH of dilution buffer depends on the type of anion-exchange solid phase and solution composition from step (a), but it is usually between pH 3 and pH 8, preferably pH 3 to pH 7, more preferably between pH 4 and pH 7.

Further, in cases where suitable conditions of contacting the solution containing the target nucleic acid(s) to the anion exchange solid phase provided in step (b) that allow binding of said target nucleic acid(s) to said solid phase are not yet met, the method of the present invention can further comprise prior to step (c) a step of equilibrating the anion exchange solid phase to a pH at which the surface of said solid phase is positively charged using an equilibration buffer.

In this step, which can be omitted in cases wherein the anion exchange solid phase is already provided with an overall positive charge, it is preferred that the pH to which the anion exchange solid phase is equilibrated is a pH that is lower than the pKa and/or the pl of said anion exchange solid phase and/or of said ligand, so that the solid phase is sufficiently positively charged. The typical pH range of the equilibration buffer is between pH 3 and pH 8, preferably between pH 4 and pH 7, but the suitable pH for equilibration depends on the properties of the ligand that is part of the solid phase. Preferentially, the solid phase is equilibrated to a pH lower than the pKa of the ligand and/or the pl of the solid phase. In a preferred embodiment, the solid phase is equilibrated to a pH value 0.5 to 4 pH units lower than the pKa of the ligand and/or the pl of the solid phase. More preferably, the solid phase is equilibrated to a pH value 0.5 to 2 pH units below the pH of the elution buffer used in step (d) of the method of the present invention. Suitable equilibration buffers are not particularly limited and are known in the art, including acetate, citrate, MES, HEPES, TRIS, bis-tris propane, and phosphate. The working buffer concentration is between 1 and 500 mM, preferably 2 to 200 mM, more preferably between 5 and 100 mM. The equilibration buffer conductivity can be adjusted with different ionic salts in a range between 1 and 100 mSi/cm, preferably between 5 and 50 mSi/cm, but not exceeding the conductivity/ionic salt concentration, at which target nucleic acid(s) do not bind anymore.

In step (d) of the method of the present invention, the nucleic acid(s) is/are eluted from the anion exchange solid phase using an elution buffer having a pH that is close to or higher than the pH at which the switch between an overall positive charge of said anion exchange solid phase to a neutral charge or a negative charge occurs. Preferably, the elution buffer has a pH that is 0.5 to 2 pH units higher than the pH at which the switch between an overall positive charge of said anion exchange solid phase to a neutral charge or a negative charge occurs. In preferred embodiments, provided that the pH of the elution buffer is close to or higher than the pH at which the switch between an overall positive charge of said anion exchange solid phase to a neutral charge or a negative charge occurs, the elution buffer can have a pH in the range of pH 4 to pH 10, in the range of pH 4 to pH 9, in the range of pH 5 to pH 8, or in the range of pH 7 to pH 8.

In further preferred embodiments, the elution buffer used in this step has a conductivity that is lower than the conductivity of the solution provided in step (a), e.g. a conductivity of 20 mSi/cm or lower (e.g. in the range of 0.2 to 20 mSi/cm), preferably 10 mSi/cm or lower (e.g. in the range of 1 to 10 mSi/cm), more preferably 5 mSi/cm or lower, more preferably 2 mSi/cm or lower, provided that said conductivity is lower than the conductivity of the solution provided in step (a). Suitable buffers for use as elution buffer in step (d) of the method of the present invention are not particularly limited and are known in the art, including acetate, citrate, MES, HEPES, TRIS, bis-tris propane, phosphate, and the like.

In specific embodiments, by way of minimizing the amount of elution buffer used for elution in step (d) of the method of the present invention, the target nucleic acid(s) can be concentrated concurrently with being purified. Further, in specific embodiments, elution in step (d) of the method of the present invention does not encompass the use of a pH gradient.

In preferred embodiments, the method of the present invention further comprises after step (c) and prior to step (d) one or more step(s) of washing the anion exchange solid phase with water or with a washing buffer whereby unbound components are washed away while the nucleic acid(s) remain(s) bound to said solid phase.

In these embodiments, 1, 2, 3, or more such additional washing steps can be performed. The washing buffer that can be used for these additional washing steps can be a buffer having a pH lower than the pH of the elution buffer used in step (d), but under conditions that that target nucleic acids do not desorb from the anion-exchange solid phase. In specific embodiments, the pH of the washing buffer is between pH 4 and pH 9, preferably between pH 5 and pH 8. Suitable washing buffers are not particularly limited and are known in the art, including acetate, citrate, MES, HEPES, TRIS, bis-tris propane, and phosphate. The washing buffer concentration is between 1 and 500 mM, preferably 2 to 200 mM, more preferably between 5 and 100 mM. Washing buffer conductivity can be adjusted with different ionic salts in a range between 0.1 and 150 mSi/cm, preferably between 0.2 and 90 mSi/cm, more preferably between 0.5 and 10 mSi/cm, but not exceeding the conductivity at which target nucleic acid(s) do not bind anymore. In specific embodiments, the washing buffer has a conductivity that is lower than the conductivity of the solution provided in step (a) to decrease the amount of remaining ionic salts while the nucleic acid(s) remain(s) bound to said solid phase. In a particular embodiment, the washing buffer can be the same as the equilibration buffer defined above.

The method of the present invention does not have any specific requirements as far as the temperature at which the method is conducted is concerned. Thus, said method, or at least steps (e) and (f) of said method, can be performed at room temperature or at a temperature in the range of 4 to 35 °C, preferably in the range of 8 to 25°C.

As used herein, the term "comprising"/"comprises" expressly includes the terms "consisting essentially of"/"consists essentially of" and "consisting of"/"consists of", *i.e.*, all of said terms are interchangeable with each other herein.

Further, as used herein, the term "about" preferably represents a modifier of the specified value of ± 10%, more preferably ± 8%, ± 6%, ± 5%, ± 4%, ± 3%, ± 2%, ± 1%, or ± 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

The present invention enables easily scalable purification/desalting of nucleic acids below pH 9, even below pH 8, or even below pH 7. The invention is realized by using an anion exchange solid phase, which 1) bears a surpass of positive charges at sample application conditions and 2), loses/decreases this (overall) positive charge in a range between pH 3 and 9 (i.e., has a pKa/pl value lower than 9, e.g. in the range of 3 to 9, preferably in the range of 4 to 7.5, more preferably in the range of 5 to 7). The switch from positive to neutral/negative surface enables the elution of bound nucleic acid in slightly alkaline, neutral, or even slightly acidic pH buffers (pH 4 to pH 10, preferably pH 4 to pH 9, pH 5 to pH 8, or 7 to pH 8) at low conductivity (< 5 mS/cm), mainly by terminating the electrostatic interaction between negatively charged nucleic acids and positively charged solid phase.

It is counterintuitive and innovative to use anion exchangers for nucleic acid purification/desalting in neutral or slightly alkaline/acidic pH, and the inventive choice of anion exchangers with pKa/pl values below pH 9 paves the way to achieving this specific goal. The whole purification/desalting step is efficiently performed at room temperature (or even lower at lower temperatures, e.g. 8 to 25 °C). The process is performed fast (seconds and minutes range) with high sample recovery and high sample integrity. The process is perfectly suitable for large-scale (up to kilograms of nucleic acids) applications in addition to smallscale (nano-and microgram range).

An important enabling feature of the invention is its platform action and extreme flexibility, wherein one solid phase and one approach (with some optimization of conditions) can be used to purify and/or desalt a broad range of nucleic acid samples in the size range from 2 kDa for small oligonucleotides up to 10 MDa or even above for large plasmid DNA. This cannot be achieved with filtration methods, dialysis, and size exclusion approaches, where materials of different pore size, cross-linking/density must be used for different sizes of nucleic acid samples. In addition, the purification/desalting mechanism behind RNA or DNA oligonucleotide-based molecules is similar, therefore the same solid phase with no need of tailoring its surface/ligands on the surface can be used for all different nucleic acid samples. In addition, the concept supports usage of many different formats, used for desalting/concentration/purification of nucleic acids, for example: Robocolumns format, spin column format, 96-well plate format, chromatographic column format, magnetic bead format, pipette tip format and the like.

None of the known methods for purifying, desalting, and/or concentrating nucleic acids combine all of the advantageous features of the method of the present invention in a single method. These advantageous features are (i) purification/desalting with simultaneous sample concentration; (ii) performing the process in three simple steps; (iii) enabling scaling up from nanograms to grams of nucleic acids; (iv) performing the process at mild conditions (neutral pH, room temperature, low shear forces); (v) no use of organic solvents; (vi) enabling nucleic acid recovery above 90%; and (vii) using one material to purify different nucleic acid modalities from 2 kDa to 10 MDa of size.

In particular, the present invention surprisingly enables a fast, high recovery, low shear stress, scalable and flexible way to perform purification/desalting of nucleic acids by binding target nucleic acid on an anion exchange solid phase (e.g. at acidic pH, pH < 7), washing away the unwanted components, such as salts (i.e., completely eliminating them) and eluting the target nucleic acid in low conductivity buffers (< 10 mSi/cm, even < 5 mSi/cm, or even < 2 mSi/cm) at neutral pH or slightly alkaline/acidic pH (pH 4 to pH 10, pH 4 to pH 9, pH 5 to pH 8, or pH 7 to pH 8). At the same time, the target molecule is eluted in a small volume, usually resulting in additional concentration of the sample compared to loading conditions. The solid phase is preferably a chromatographic support, more preferably a support, where mass transfer is based on convection, such as monoliths, membranes, or fibers. Such chromatographic supports enable low shear-force conditions.

There are three more additional features that this invention enables. Firstly, the same technical set-up can be used as only a desalting step or as a dual-action process, where purification of nucleic acids is performed before their desalting, which is e.g. facilitated by the addition of one or two washing steps in the process method. In this way, target nucleic acid can be purified from contaminating nucleic acids, proteins, and/or lipids. Secondly, the present invention can also be applied to purify nucleic acids from organic solvents (e.g., acetonitrile, ethanol, 1-propanol, 2-propanol), which are used in purification of nucleic acids, especially on laboratory scale. Another enabling feature of this invention is the possibility to also add desired components (e.g. stabilizing agents) into the final solution of target nucleic acids, practically enabling a buffer exchange. This can be achieved simply by adding the desired components into a wash buffer or even into a loading buffer, as well as into the elution buffer.

Previous to the present invention, a person skilled in the art would not have considered using an anion exchanger around neutral pH for desalting to conductivities below e.g. 5 mSi/cm. Instead, a person skilled in the art would have performed ion exchange chromatography for capture and purification of nucleic acids in salt gradient (or HIC (Hydrophobic Interaction Chromatography) approach for polishing, also salt gradients), with nucleic acid eluting in a high salt buffer. Next, the person skilled in the art would have used another method for desalting (and/or buffer exchange/concentrating) the eluate (e.g. TFF). Alternatively, one could have used a high pH buffer (pH > 9.0) with low conductivity for elution of nucleic acids from weak anion exchangers (e.g. diethylamino-modified), but high pH is known to negatively influence the nucleic acid integrity. Employing a low conductivity (< 5 mS/cm) buffer with pH < 9.0 does not result in the elution of nucleic acids from standard strong and weak anion exchange solid phases (quaternary amine, tertiary amine) or even multimodal PrimaS from Sartorius BIA Separations d.o.o. as these solid phases are still positively charged at pH values below pH 9.

For single-stranded RNA (ssRNA), desalting can be achieved by an affinity chromatography approach using for example an oligo-deoxythymidine (dT) functionalized solid phase for binding poly-adenylated mRNA. The elution of mRNAfrom such solid phase can be achieved at low conductivity in dH₂O/ddH₂O or low-conductivity buffers. This approach however is sequence-dependent and one solid phase cannot be used for desalting of different types of nucleic acids (e.g. ssRNA, double-stranded RNA (dsRNA), dsDNA, oligonucleotides), which is enabled by the method of the present invention.

The present invention is a process in which the first step employs a specific positively charged solid phase equilibrated to mildly or moderately acidic conditions to enable binding, concentration and removing the buffer components and put nucleic acids in a chemical environment suitable for further processing. This first step may be followed by one or more washing steps. In the third step, an elution buffer is applied, enabling the release of adsorbed nucleic acids into a solution. The pH and conductivity of the elution buffer need to be selected properly to achieve as high as possible concentration of eluted target nucleic acids with as high as possible recovery. It is recommended to use chromatographic solid phases, where mass transfer is based on convection (monoliths, membranes, fibers), because of their known advantages over porous beads when working with large biomolecules, such as plasmid DNA, messenger RNA, and the like. An example of an appropriate solid phase is CIM monolith chromatographic phase or Sartobind membrane adsorber modified with e.g. a ligand bearing imidazole or pyridine groups (e.g. CIM histamine). The CIM and Sartobind columns are scalable, and the same method can be applied to desalt nucleic acids in a microgram range up to gram an even decagram range in a single run.

Additionally, the following conditions can be met with the present invention:
(i) Nucleic acids can be bound to the specific solid phase at pH <9, pH <8, pH < 7, pH < 6 or pH < 5.
(ii) The starting sample (prior to sample preparation) to be loaded on the solid phase can contain target nucleic acid(s) and one or more ionic salts. Examples of ionic salts include organic or inorganic salts (for example: NaCl, KCl, Na-acetate, Na-phosphate, MgCl₂, CaCl₂, pyrophosphate, ammonium sulphate, EDTA) at concentrations of ≥ 0.01 M, ≥ 0.001 M, or even ≤ 0.001 M. Alternatively, or additionally, the starting sample (prior to sample preparation) to be loaded on the solid phase can contain target nucleic acid(s) and one or more of the following components: organic solvents (e.g. acetonitrile, formamide, formaldehyde, ethanol, 1- propanol, 2-propanol), surfactants, organic components (e.g. urea, guanidinium). Alternatively, or additionally, the starting sample (prior to sample preparation) to be loaded on the solid phase contains target nucleic acid and one or more of the following biomolecules other than the target nucleic acid: nucleotides (including nucleotide monophosphates, nucleotide diphosphates or nucleotide triphosphates), proteins, enzymes (e.g. DNase, RNase, pyrophosphatase, RNA-polymerase), lipids, carbohydrates, unwanted nucleic acids (e.g. oligonucleotides, template DNA, rRNA, small RNA).
(iii) Concentration of nucleic acids can be achieved with the method of the present invention when working with low concentration starting material (e.g. < 100 µg/mL DNA or RNA).

The present invention provides desalting and purification in one purification/separation step, which would otherwise need an additional processing step. The present invention therefore intensifies the downstream processing by combining two distinctive features using the same solid phase, i.e., purification followed by a desalting/buffer exchange in a single step. It is counterintuitive to combine anion-exchange chromatography for simultaneous purification and desalting. In particular, a person skilled in the art would firstly perform purification of nucleic acids on anion exchange solid phase, followed by desalting of the eluate by any of known desalting methods. In the present invention the conditions for binding nucleic acids on the anion exchange solid phase is chosen in a way that some impurities (if present in the sample) can be selectively eluted from the solid phase in an additional washing step, while the target molecules remain bound to the solid phase. The first washing step can be performed using a buffer with increased pH or increased ionic salt concentration or increase in organic solvent etc., to selectively desorb only the impurities. The impurities could be from different groups, such as proteins, endotoxins, lipids, but also nucleic acids. Next, a second washing step is performed with a low conductivity buffer at a pH below the pH of the elution buffer to remove the components of the buffer used for elution of impurities and to establish low conductivity (thereby desalting of the initial sample). In the last step elution buffer is applied, enabling the release of adsorbed target nucleic acids into solution. The pH and conductivity of the elution buffer need to be selected to achieve desired desalting with as high as possible concentration of eluted target nucleic acids with as high as possible recovery of said nucleic acid.

The methods of the present invention can be used to purify/desalt samples comprising a variety of nucleic acids, including, for example, single-stranded nucleic acids and double-stranded nucleic acids. Such nucleic acids include deoxyribonucleic acids (DNA) (e.g., genomic DNA, cDNA, chromosomal DNA, plasmid DNA), ribonucleic acids (RNA) (e.g., messenger RNA (mRNA), transfer RNA (tRNA), ribosomal RNA (rRNA)), deoxyribonucleic acid-ribonucleic acid hybrids (DNA-RNA hybrids) and nucleic acids comprising deoxyribonucleotides and/or ribonucleotides. The conformation of the nucleic acids can be linear (e.g. linear DNA, mRNA, ssRNA) or circular/covalently closed (e.g. circular plasmid DNA, circular RNA). The methods of the present invention are applicable not only to nucleic acids which are comparatively large in size (e.g., chromosomal DNA, genomic DNA) but also to those which are comparatively small in size (e.g., oligonucleotides). In one embodiment, the methods of the present invention are used to purify/desalt single-stranded and/or double-stranded oligonucleotides. The methods of the present invention can further be used for naturally occurring nucleic acids, for example, naturally occurring nucleic acids which are isolated from an organism, including mammals and in particular, humans. Alternatively, the methods of the present invention can be used for modified nucleic acids, such as, for example, a nucleic acid which was isolated from an organism, and then modified to alter (e.g., add, delete, or change) one or more nucleotides; the methods of the present invention can also be used for recombinantly-produced nucleic acids. The methods of the present invention can also be used for synthetic nucleic acids; that is, nucleic acids which are prepared by artificial means, rather than isolated from an organism. For example, the synthetic nucleic acid can be produced using an automated synthesizer. Examples of synthetic or modified nucleic acids include nucleic acids which contain nucleotides or nucleic acid analogs that are normally not found in nature, for example, phosphorothioates, protein-nucleic acids, protein-nucleic acid hybrids and synthetic nucleic acids comprising at least one modified nucleotide (e.g., a dideoxynucleotide, a biotinylated nucleotide, an amine modified nucleotide, an alkylated nucleotide, a fluorophore labeled nucleotide, a radiolabeled nucleotide). Nucleic acids (both naturally occurring and synthetic) can be isolated or produced using a variety of means, for example enzymatic reactions, wherein the nucleic acid is an *in vitro* transcription reaction product, a polymerase chain reaction product or a minisequencing product. In one embodiment, the methods of the present invention can be used for liquid samples containing more than one type of nucleic acid (e.g., a liquid sample containing a naturally occurring nucleic acid and a modified nucleic acid; a sample containing a single stranded and double stranded nucleic acid; a sample containing an RNA and a DNA nucleic acid, a sample containing different types of RNA, for example transfer RNA and ribosomal RNA etc.).

The following process steps may be used, to achieve purification/desalting of DNA or RNA:
1) A liquid (or solid) sample containing target nucleic acid and optionally one or more ionic salts is provided. The conductivity of liquid samples can be ≥ 5 mSi/cm and the pH in the range of 2 to 13. Examples of ionic salts include organic or inorganic salts containing one or more of the ions: Na⁺, K⁺, Cl⁻, Li⁺, acetate, phosphate(V), Mg²⁺, Ca²⁺, Mn²⁺, pyrophosphate, ammonium, sulphate(VI), EDTA, citrate, thiocyanate, guanidinium (for example: NaCl, KCl, Na-acetate, Na-phosphate, MgCl₂, CaCl₂, Na-/K-pyrophosphate, ammonium sulphate, EDTA, guanidinium hydrochloride, guanidinium thiocyanate) at concentrations of ≥ 0.01 M, even ≥ 0.001 or even ≤ 0.001 M.
2) Alternatively, or additionally, the starting sample (prior to sample preparation) to be loaded on the solid phase contains target nucleic acid and one or more of undesired organic components (at content ≥ 0.01 % (or even ≥ 0.001 %). The conductivity of such samples is not limited to > 5 mSi/cm, but can be lower. Examples of organic components are acetonitrile, formamide, formaldehyde, ethanol, 1-propanol, 2-propanol, urea, surfactants (poloxamer, tween, triton), sorbitol, saccharose, spermine, spermidine.
3) Alternatively, or additionally, the starting sample (prior to sample preparation) to be loaded on the solid phase contains target nucleic acid, one or more ionic salts and one or more of the following biomolecules other than the target nucleic acid: nucleotides (including nucleotide monophosphates, nucleotide diphosphates or nucleotide triphosphates), proteins, enzymes (e.g. DNase, RNase, pyrophosphatase, RNA-polymerase), lipids, carbohydrates, other non-target nucleic acids (e.g. oligonucleotides, template DNA, rRNA, small RNA).
4) A solid phase bearing anion exchanging groups with pKa/pl ≤ 9.0 and ≥ 3.0 is provided. Example of such solid phase is monolith CIM histamine or CIM mercaptopyridine from Sartorius BIA Separations d.o.o.
5) The solid phase is equilibrated to a pH, where its surface is positively charged, which enables binding of negatively charged nucleic acids. For example, 50 mM acetate buffer, pH 5.0 could be used in combination with a CIM histamine column.
6) The sample is prepared by adjusting its pH to the same pH value (or below) as the solid phase is equilibrated at. The conductivity of the sample may need to be adjusted to enable binding of the nucleic acid at desired pH.
7) The sample is contacted to the solid phase with target nucleic acid binding to the solid phase. For example, this can be done by pumping an adjusted sample solution through a chromatographic column containing the selected solid phase with the appropriate pKa/pl.
8) The solid phase is then washed with equilibration buffer to wash away unbound components, including ions, detergents, organic solvents, salts, etc., while target nucleic acid remains bound to the solid phase.
9) Next, the solid phase can be washed with a second low conductivity buffer with pH below elution pH or with pH the same as equilibrated pH to eliminate unwanted binding buffer components ions/salt, which do not bind to the solid phase. The second wash buffer can be skipped if equilibration buffer suits the aforementioned requirements.
10) Additional wash steps can be added to achieve an additional purification of target nucleic acid (e.g. from other nucleic acids, proteins, lipids, carbohydrates, oligonucleotides) as described below.
11) Lastly, the bound nucleic acid is eluted in a low conductivity buffer with pH in the range of 5.0 to 9.0. For example, 50 mM TRIS buffer, pH 8.0, could be used in combination with a CIM histamine column.
12) In one embodiment, the elution buffer can contain a salt/component to be buffer exchanged into the nucleic acid solution. (This component can also be included in the second wash buffer to achieve an effective buffer exchange).

The present invention can be used to achieve a combined purification and desalting of a nucleic acid in a single run. Such example is desalting and purification of linearized plasmid DNA/DNA after endonuclease/linearization treatment. The method is applied to separate nucleic acid from proteins (e.g. enzymes) present in the reaction mixture, as well as to desalt the sample and eliminate the ions/components present in the endonuclease reaction buffer. Some enzymes will not bind to the anion exchange solid phase when contacted at pH 5.0, whereas plasmid DNA will bind to the solid phase. In this case enzymes can be easily removed as described above. Some enzymes will bind to the anion exchange solid phase when contacted at pH 5.0. An additional wash step (for example a high salt wash step at 50 mS/cm at pH 5.0 or a low pH step at e.g. pH 3.0) may be applied to remove these enzymes from the solid phase. Additionally, loading can be performed at lower pH (e.g. pH 4.0 or even 3.0) to prevent enzymes from binding to the solid phase and enable their easy removal.

Another example of combined purification and desalting is purification of mRNA/ssRNA/RNA after DNase treatment to remove leftover template DNA. In this case the present invention enables removal of enzymes, DNA fragments/nucleotides as well as reaction buffer components.

Some application fields of the methods of the present invention include the following:
1) Desalting DNA, RNA, or oligonucleotides and/or removing organic solvents as a step in industrial purification, e.g. after capture/polishing/purification steps using anion exchange chromatography, hydrophobic interaction chromatography or reversed-phase chromatography.
2) Desalting, concentrating and/or purifying nucleic acids (RNA and DNA) from enzymes and buffer components after enzymatic reactions (*in vitro* transcription, DNase treatment, RNase treatment, protease treatment, polymerase chain reaction).
3) Desalting and concentration of nucleic acids before electrophoretic analysis.
4) Desalting and concentration of nucleic acids before mass spectrometry analysis.
5) Desalting and concentration of nucleic acids in high throughput manner for diagnostic applications, for example using 24-well, 96-well, 386-well plate format, RoboColumn^{®} format, etc.
6) Capturing (desalting, concentrating, and purifying) plasmid DNA from neutralized cell lysate, which is an essential step in the purification of plasmid DNA, indispensable molecule in the whole gene therapy and vaccination industry.

The figures show:
Figure 1:
   Chromatogram showing the desalting of model plasmid DNA (pFIX5). Binding and salt removal is achieved by contacting CIM PrimaH monolith with the pDNA sample containing 0.4 M NaCl at pH 5. Desalting was performed by changing the buffer to 20 mM TRIS, pH 8, where bound pDNA was efficiently recovered at 3 mSi/cm.
Figure 2:
   Electropherograms of samples containing eGFP and mFIX mRNA, desalted with Amicon ultracentrifugation filter (A) or CIM PrimaH monolith (B) using the procedure of the present invention.
Figure 3:
   Electropherograms of samples containing eGFP and mFIX mRNA, desalted with Amicon ultracentrifugation filter (A) or CIM PrimaH monolith (B) using the procedure of the present invention.
Figure 4:
   Desalting and concentrating of 13 mg of pHELP11.6 kbp-large plasmid using a CIMmultus PrimaH column with 8 mL bed volume. The following steps are visible from the chromatogram: sample loading from 0 to 27 minutes, washing from 27 to 30 minutes, elution after 30 min.
Figure 5:
   Desalting, concentration (and purification) of plasmid DNA from clarified bacterial cell lysate. (A) Chromatogram of the elution step; the gray band corresponds to the collected elution fraction. (B) CIMac pDNA analytical chromatograms demonstrating the profile of the diluted lysate (1_LOAD_Diluted lysate), flow-through fraction (2_PrimaH_FT) and elution fraction (3_PrimaH_E). Red band corresponds to the elution time of bacterial RNA and the blue band to the elution time of plasmid DNA.

The present invention will be further illustrated by the following example without being limited thereto.

### Examples

### Example 1:

### Desalting of plasmid DNA after chromatographic purification (e.g. after anion-exchange or HIC chromatography).

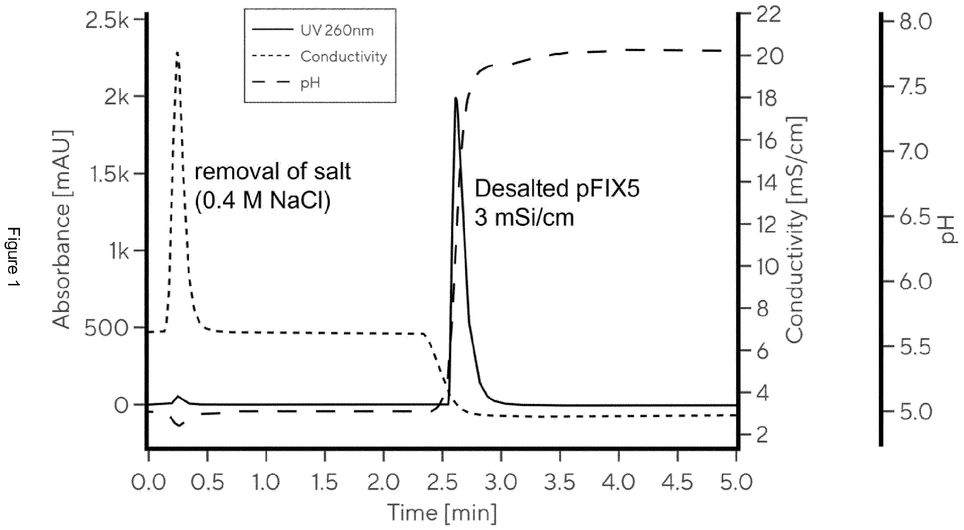

Plasmid pFIX5 was firstly chromatographically purified from clarified bacterial lysate using a CIMmultus DEAE column (Sartorius BIA Separations d.o.o.) and eluted in 50 mM Tris-HCl, 0.8 M NaCl, pH 7.2 at a concentration of 640 µg/mL. pFIX5 was then desalted using a CIM PrimaH anion exchange column (Sartorius BIA Separations d.o.o.) as an alternative to an established TFF procedure. DEAE eluate containing pFIX5 was diluted 1:1 in 50 mM citrate, pH 5.0 (buffer conductivity 6 mS/cm) to obtain a loading sample (still containing approximately 0.4 M NaCl and the conductivity of 35 mSi/cm). 0.1 mL CIMmic PrimaH disk was equilibrated with 50 mM citrate, pH 5.0. The sample was contacted with the solid phase via an HPLC system at a flowrate of 1 mL/min, whereas the pFIX5 plasmid DNA bound to the solid phase. Ionic salts (NaCl) however passed through the column unbound in a wash with 50 mM citrate, pH 5.0 (visible as the flow-through conductivity peak in Figure 1). Bound pFIX5 was later eluted with elution buffer 50 mM Tris-HCl, pH 8.0, where the plasmid eluted in the buffer with conductivity of approximately 3 mS/cm to achieve desalting (Fig. 1).

### Example 2:

### Desalting of mRNA after HIC purification.

mRNA can be purified using a hydrophobic interaction chromatography approach in a descending NaCl gradient on CIMmultus C4 HLD columns (Sartorius BIA Separations d.o.o.). The mRNA elutes in a buffer containing significant amounts of NaCl, for example 50 mM Tris-HCl, 0.4 M NaCl, pH 8.0. The method of the present invention was applied to desalt such a sample with CIM PrimaH.

A 0.05 mL CIM PrimaH column was cleaned with 10 CV (column volumes) of 1 M NaOH and equilibrated first with 20 CV of 100 mM acetate buffer, 1 M NaCl pH 5.0 and next with 20 CV of 50 mM citrate buffer, pH 5.0. A process sample of eGFP mRNA in 50 mM Tris, 0.4 M NaCl, pH 8.0 was diluted five times with 50 mM citrate buffer, pH 5.0 and 200 µL of such sample (containing approximately 5 µg of mRNA) was contacted to the equilibrated CIM PrimaH column, whereby mRNA bound to the column. NaCl salt did not bind to the column and was washed away firstly by 10 CV of 50 mM citrate buffer, pH 5.0 and next with 10 CV of purified water to completely eliminate the present salts. Air was pushed through the column to remove residual solution in the monolith. eGFP mRNA was next eluted with 100 µL of 10 mM Tris-HCl buffer, pH 8.0. eGFP mRNA was thus desalted of the 0.4 M NaCl present in the initial sample.

### Example 3:

### Desalting and purification of linear plasmid DNA from proteins and buffer components after plasmid DNA linearization.

Linearization of plasmid DNA is performed in appropriate buffers for the specific endonuclease used, typically containing Mg²⁺ (and/or other divalent cations, e.g. Ca²⁺, Mn²⁺) and buffer components. The method of the present invention enables a combined desalting (to remove free Mg²⁺) and purification (to remove the endonuclease protein itself) step to obtain a pure linear DNA sample using CIM PrimaH.

A linearization reaction of a 6.5 kbp plasmid was performed with endonuclease Notl-HF (New England Biolabs, Ipswitch, MA, USA) in rCutSmart buffer (New England Biolabs).

A 0.1 mL CIM PrimaH column was first equilibrated with 50 mM citrate buffer, pH 5.0. After linearization, the reaction mixture containing the target plasmid, ionic Mg²⁺ salts and the endonuclease enzyme was diluted approximately 1:1 with 50 mM citrate buffer, pH 5.0 and contacted with the equilibrated solid phase, whereas only plasmid DNA bound to the solid phase. Ionic Mg²⁺ salts, the endonuclease enzyme and other linearization buffer components were removed from the solid phase by washing it with 50 mM citrate buffer, pH 5.0. Next, a wash with ultrapure water was performed to remove the remaining ionic salts present in the 50 mM citrate buffer, pH 5.0. Finally, plasmid DNA was eluted with elution buffer 10 mM Tris-HCl, pH 8.0 to recover the plasmid in a low-conductivity solution.

### Example 4:

### Desalting and purification of mRNA from proteins, leftover DNA fragments and buffer components after DNase treatment.

DNase I treatment can be used to degrade template DNA present in ssRNA/mRNA preparations, e.g. after IVT (*in vitro* transcription) reaction. The reaction is performed in specific buffers, typically containing Mg²⁺ (and/or other divalent cations, e.g. Ca²⁺, Mn²⁺) and produces leftover nucleotides/DNA fragments. The method of the present invention again enables a combined desalting (to remove divalent cations) and purification (to remove the DNase I protein and nucleotides/degradation products) step to obtain a pure RNA sample using CIM PrimaH.

DNase I treatment was performed after *in vitro* transcription of Cas9 mRNA from its template DNA. A 0.1 mL CIM PrimaH column was first equilibrated with 50 mM citrate buffer, pH 5.0. After DNase I treatment, the reaction mixture containing the target mRNA, ionic Mg²⁺ salts, DNase I enzyme, IVT enzymes and nucleotides was diluted approximately ten times with 50 mM citrate buffer, pH 5.0 and 100 µL of such solution was contacted with the equilibrated solid phase, whereas only target mRNA bound to the solid phase. Ionic Mg²⁺ salts, the enzymes, nucleotides, and reaction buffer components were removed from the solid phase by washing it with 10 CV 50 mM citrate buffer, pH 5.0. Next, a wash with ultrapure water was performed to remove the remaining ionic salts present in the 50 mM citrate buffer, pH 5.0. Finally, eGFP mRNA was eluted with elution buffer 50 mM Tris-HCl, 10 mM EDTA, pH 8.0 to recover the pure mRNA without unwanted Mg²⁺ salts present. Furthermore, EDTA was included in the final mRNA solution to prevent RNase-catalysed degradation of mRNA.

### Example 5:

### Removal of organic solvents from nucleic acid preparations.

Purification of nucleic acid can be achieved with reverse phase chromatography (for example using CIMmultus styrene-divinyl benzene (SDVB) solid phase), where target nucleic acid is eluted in a buffer containing significant amounts of organic solvents (e.g. acetonitrile at concentrations ≥ 1 %). The method of the present invention enables removal of organic solvents by binding the target nucleic acid to the solid phase, washing away the organic solvent, and eluting the target RNA in water-based buffer.

Proprietary mFIX mRNA (4000 nt) was purified of impurities with ion-pair reverse phase chromatography using CIMmultus SDVB column, resulting in mRNA solution in buffer 100 mM triethylammonium acetate (TEAA), 10%(v/v) acetonitrile pH 7. A 0.1 mL CIM PrimaH column was cleaned with 10 CV of 1 M NaOH and equilibrated first with 20 CV of 100 mM acetate buffer, 1 M NaCl pH 5.0 and next with 20 CV of 50 mM citrate buffer, pH 5.0. The process sample of mFIX mRNA in 100 mM triethylammonium acetate, 10%(v/v) acetonitrile pH 7 was diluted ten times with 50 mM citrate buffer, pH 5.0 and 500 uL of such sample was contacted to the equilibrated CIM PrimaH column, whereby mRNA bound to the column. TEAA and acetonitrile did not bind to the column and were washed away firstly by 20 CV of 50 mM citrate buffer, pH 5.0 and next with 20 CV of purified water. Air was pushed through the column to remove residual solution in the monolith. mFIX mRNA was next eluted with 200 µL of 10 mM Tris-HCl buffer, pH 8.0.

Organic solvent acetonitrile was thus removed from the mFIX mRNA, which was finally obtained in a water-based buffer.

### Example 6:

### Desalting of mRNA samples before electrophoretic analysis (agarose gel electrophoresis, capillary electrophoresis).

Salts in nucleic acid samples can disturb their electrophoretic analysis and cause seeming deviations in their relative size. Nucleic acids containing high salt concentrations are typically desalted prior to electrophoretic analysis, using for example ultracentrifugation spin columns, precipitation, or solid phase extraction with silica column. The method of the present invention enables desalting of small amounts (microgram range) of nucleic acids prior to electrophoretic analysis and offers a platform way to desalt different kinds of samples.

A sample containing a mixture of eGFP mRNA (995 nt) and mFIX mRNA (4000 nt) in buffer 20 mM Tris-HCl, 0.2 M NaCl pH 7.5 was provided for analysis by capillary gel electrophoresis under denaturing conditions.

A 0.05 mL CIM PrimaH column was cleaned equilibrated with 50 mM citrate buffer, pH 5.0. The sample containing eGFP and mFIX mRNA in 20 mM Tris-HCl, 0.2 M NaCl, pH 7.5, was diluted five times with 50 mM citrate buffer, pH 5.0 and 200 µL of such sample (containing approximately a total of 10 µg of mRNA) was contacted to the equilibrated CIM PrimaH column, whereby mRNA bound to the column. Excess NaCl salt was washed away firstly by 10 CV of 50 mM citrate buffer, pH 5.0 and next with 10 CV of purified water to completely eliminate the present salts. Air was pushed through the column to remove residual solution in the monolith. mRNA was next eluted with 100 µL of 10 mM Tris-HCl buffer, pH 8.

As a reference, the same amount of the initial sample was buffer exchanged into ddH₂O using Amicon Ultra-0.5 Centrifugal Filter Unit (10 kDa MWCO, Merck) following the producer's protocol.

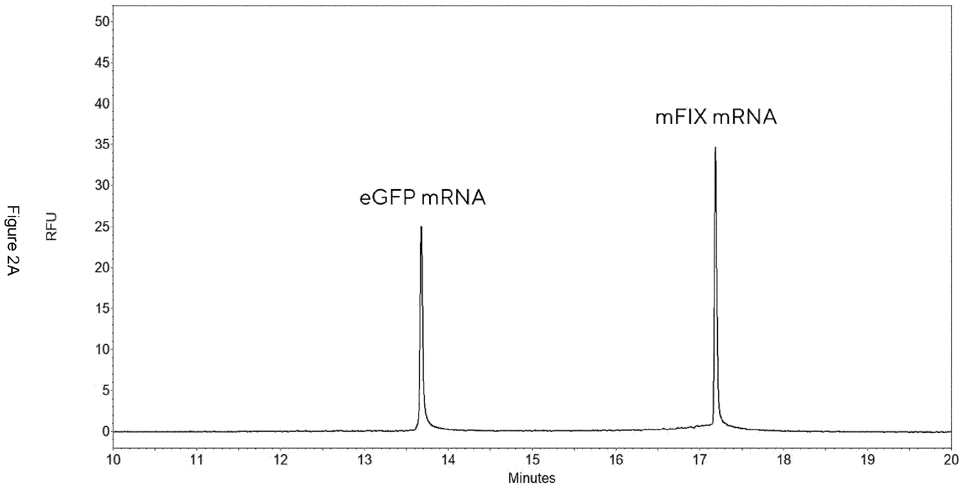

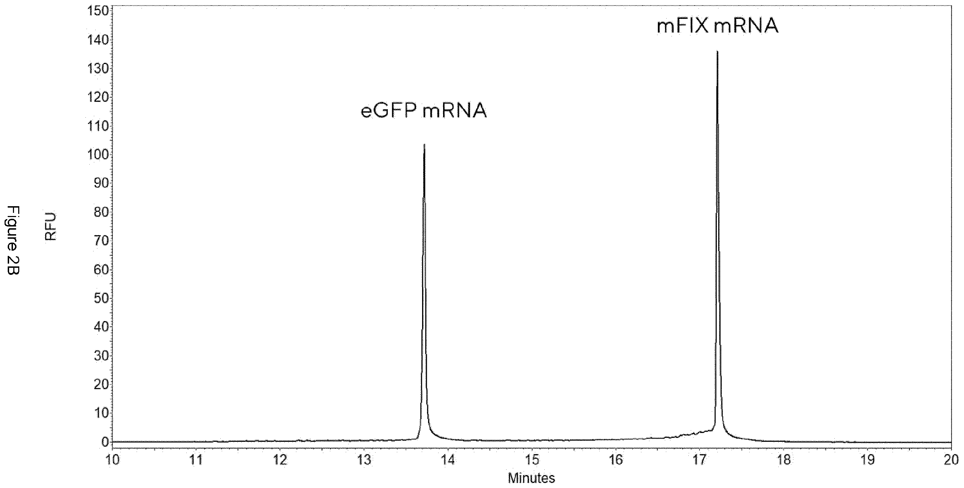

Both samples were analysed by capillary gel electrophoresis and the electropherograms are shown in Figure 2. Comparison of the time-corrected relative peak areas showed the same profile (43% of the total area belonged to eGFP mRNA and 57% to mFIX mRNA) of centrifugal filter desalted and CIM PrimaH desalted samples, demonstrating that the CIM PrimaH desalting is efficient and does not influence the sample composition. The method of this invention could therefore be appropriate for sample preparation prior to electrophoretic analysis similar to centrifugal filters.

### Example 7:

### Desalting of RNA samples of various size (200-6000 nt)

To demonstrate that the method of the present invention enables desalting of various sizes of RNA molecules, RiboRuler High Range RNA Ladder (Thermo Scientific), containing RNA of sizes in the range of 200-6000 nt, was desalted with CIM PrimaH using the established method.

A 0.1 mL CIM PrimaH column was equilibrated with 50 mM citrate buffer, pH 5.0. 10 µL of RiboRuler High Range RNA Ladder was diluted ten times with 50 mM citrate buffer, pH 5.0 contacted to the equilibrated CIM PrimaH column, whereby ssRNA bound to the column. Column was washed with 10 CV of 50 mM citrate buffer, pH 5.0 and next with 10 CV of purified water. Air was pushed through the column to remove residual solution in the monolith. ssRNA was next eluted with 150 µL of 10 mM Tris-HCl buffer, pH 8.

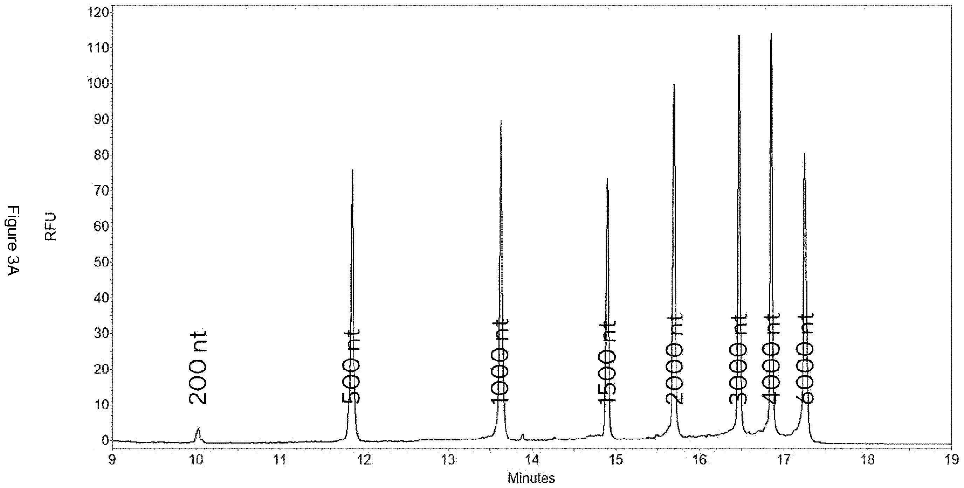

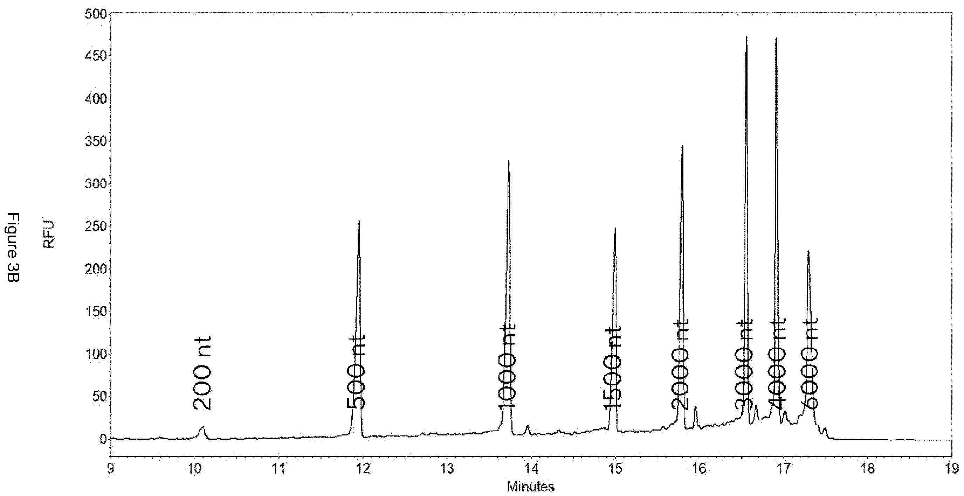

The desalted sample as well as the unprocessed RiboRuler High Range RNA Ladder were buffer exchanged into ddH₂O using Amicon Ultra-0.5 Centrifugal Filter Unit (10 kDa MWCO) and analysed by capillary gel electrophoresis under denaturing conditions and the electropherograms are shown in Figure 3.

Comparison of the time-corrected relative peak areas showed similar content of all present RNA species demonstrating that CIM PrimaH desalting is appropriate for a broad range of differently sized RNA molecules. This is also true for DNA molecules but is not shown here.

### Example 8:

### Desalting and concentrating large amounts of plasmid DNA (preparative application of the present invention)

Previous examples showcased the applicability of the method of the present invention for desalting of relatively small amounts (µg range) of nucleic acids. Here we demonstrate the easy scalability of the approach by desalting and concentrating approximately 13 mg of 11.6 kbp-large pDNA with 8 mL CIMmultus PrimaH column using the method of the present invention.

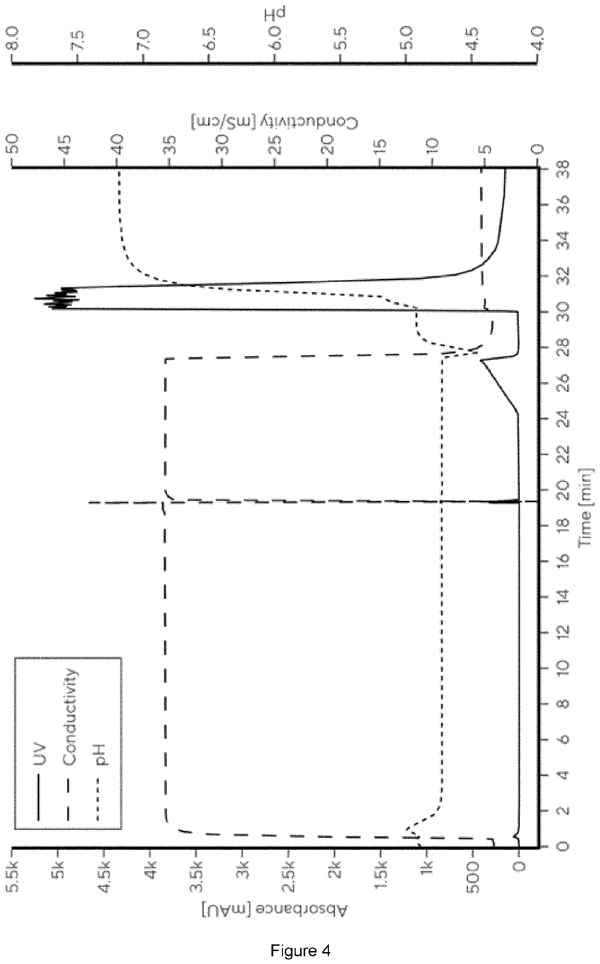

A sample containing 11.6 kbp-large plasmid at a concentration of approximately 70 µg/mL in buffer 50 mM Tris-HCl, 0.8 M NaCl, 10 mM EDTA, pH 7.2 was provided. CIMmultus PrimaH column with 8 mL bed volume was used for desalting and concentrating, while the processing was performed using an ÄKTA pure 150 M (Cytiva, Marlborough, MA, USA) system at a flowrate of 16 mL/min (2 CV/min). Prior to loading, the column was cleaned with 10 CV of 1 M NaOH, followed by equilibration with 10 CV 100 mM acetate buffer, 1 M NaCl, pH 5.0 and 20 CV of 50 mM acetate buffer, 10 mM EDTA, pH 5.0 which was used as binding buffer. The initial sample was diluted 1:1 with 100 mM acetate buffer, 10 mM EDTA, pH 5.0 to establish binding conditions. The concentration of plasmid in the loading sample was 34 µg/mL, while loading sample conductivity and pH were approximately 35 mS/cm and 5.0, respectively. The sample was applied to the column at a flowrate of 16 mL/min until breakthrough, followed by a 5 CV wash with 50 mM acetate buffer, 10 mM EDTA, pH 5.0 to remove excess salt (visible as a dip in conductivity at 27 min). Finally, 11.6 kbp-large plasmid was eluted with 50 mM Tris-HCl, 10 mM EDTA, pH 7.2, the elution volume was approximately 4.5 CV (36 mL), plasmid elution recovery was 90% and the concentration of plasmid in the elution fraction was approximately 350 µg/mL (Fig. 4). Including column preparation time, the sample was desalted in approximately 1h, while we also achieved a five time plasmid concentration compared to the initial sample.

### Example 9: Desalting, concentration (and purification) of plasmid DNA from clarified bacterial cell lysate

Clarified bacterial lysate typically contains a target plasmid DNA and contaminants such as residual proteins, bacterial RNA, sodium dodecyl sulphate, residual membrane components, and salts, typically EDTA, CaCl₂, CH₃COOK and NaCl.

A clarified *E*. *coli* lysate with a pH of 4.6 and conductivity of approximately 140 mS/cm was provided. The goal was to desalt, concentrate and purify target plasmid DNA.

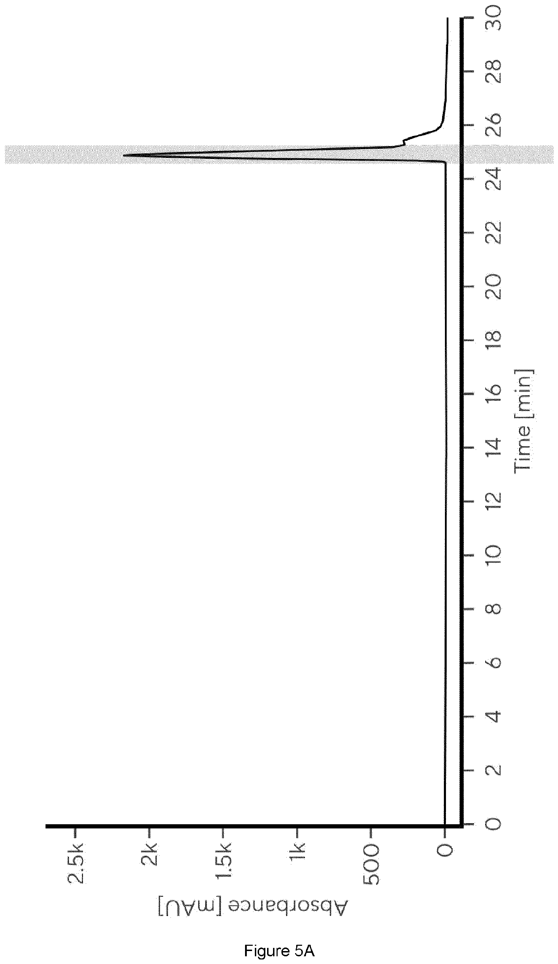

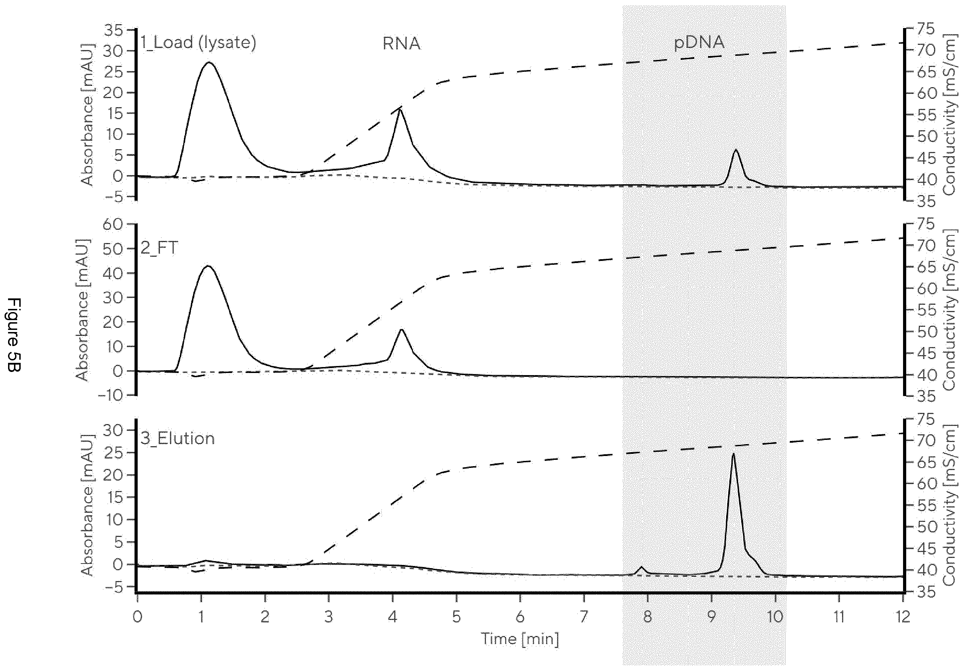

A 0.1 mL CIM PrimaH column was provided and equilibrated with 50 mM citrate buffer, pH 5.0. The provided lysate was diluted four times with purified 50 mM MES buffer, pH 5.0 to reduce the conductivity of the sample and enable binding of plasmid DNA to the solid phase; the conductivity of the loading sample was 55 mS/cm. 10 mL of the diluted lysate was manually applied to the 0.1 mL CIM PrimaH column using a syringe and the flow-through fraction was collected. The column was next mounted to a PATfix HPLC system to monitor the elution via UV detection. Unbound components were washed with 50 CV of 50 mM citrate buffer, pH 5.0, followed by a linear gradient elution into 50 mM Tris-HCl, pH 8.0 over 300 CV. The main elution peak was collected for analysis as demonstrated in Figure 5A. Collected fractions were analysed by CIMac pDNA analytical method as reported previously (Fig. 5B). Analytics confirmed that the collected elution fraction contained only plasmid DNA with no RNA or protein contaminants present.

## Claims

1. A method for purifying one or more target nucleic acid(s), comprising the steps of:
(a) providing a solution containing the target nucleic acid(s);
(b) providing an anion exchange solid phase, **characterized in that** a switch between an overall positive charge of said anion exchange solid phase to a neutral charge or a negative charge occurs in a pH range of between pH 3 and pH 9;
(c) contacting the solution containing the target nucleic acid(s) to said anion exchange solid phase under conditions that allow binding of said target nucleic acid(s) to said solid phase; and
(d) eluting the nucleic acid(s) from the anion exchange solid phase using an elution buffer having a pH that is close to or higher than the pH at which the switch between an overall positive charge of said anion exchange solid phase to a neutral charge or a negative charge occurs.

2. The method of claim 1, wherein the nucleic acid is selected from the group consisting of genomic DNA, cDNA, chromosomal DNA, plasmid DNA, DNA oligonucleotides, messenger RNA (mRNA), transfer RNA (tRNA), ribosomal RNA (rRNA), small interfering RNA (siRNA), circular RNA, self-amplifying RNA, RNA oligonucleotides, and mixtures thereof.

3. The method of claim 1 or claim 2, wherein purifying the one or more target nucleic acid(s) comprises the removal of
(i) ionic salts, and/or
(ii) undesired organic compounds, and/or
(iii) undesired organic solvents, and/or
(iv) biomolecules other than the target nucleic acid(s),
that may be present in the solution provided in step (a).

4. The method of any one of claims 1 to 3, wherein the solution provided in step (a) contains
(i) one or more ionic salt(s), and/or
(ii) one or more undesired organic compound(s), and/or
(iii) one or more undesired organic solvent(s), and/or
(iv) one or more biomolecule(s) other than the target nucleic acid.

5. The method of claim 4, wherein the ionic salt(s) is/are selected from the group consisting of organic or inorganic salts containing one or more ion(s), selected from the group consisting of Na⁺, K⁺, Cl⁻, Li⁺, acetate, phosphate(V), Mg²⁺, Ca²⁺, Mn²⁺, pyrophosphate, ammonium, sulphate(VI), EDTA, citrate, thiocyanate, nitrate (V), and guanidinium.

6. The method of any one of claims 1 to 5, wherein the anion exchange solid phase comprises one or more ligand(s), said ligand(s) comprising one or more functional group(s), wherein at least one of said functional group(s) has a pKa of between pKa 3 and pKa 9.

7. The method of claim 6, wherein said functional group(s) is/are selected from the group consisting of substituted aliphatic amines and nitrogen-containing heterocycles.

8. The method of claim 7, wherein
(i) said substituted aliphatic amines are selected from the group consisting of glucosamine and tricine, and/or
(ii) said nitrogen-containing heterocycles are selected from the group consisting of purines, pyrimidines, imidazoles, pyridines, diazoles, triazoles, morpholines, and tetrazoles.

9. The method of any one of claims 1 to 8, wherein the anion exchange solid phase and/or a ligand comprised in said anion exchange solid phase has a pl of 8.5 or less.

10. The method of any one of claims 1 to 9, wherein said anion exchange solid phase is a chromatographic solid phase, selected from the group consisting of chromatographic monoliths, chromatographic membranes, chromatographic beads, chromatographic fibers, and chromatographic columns comprising a solid stationary phase.

11. The method of any one of claims 1 to 10, wherein the elution buffer used in step (d) has a conductivity that is lower than the conductivity of the solution provided in step (a).

12. The method of any one of claims 1 to 11, further comprising prior to step (c) a step of adjusting the solution provided in step (a) to a pH and conductivity that allow binding of said target nucleic acid(s) to said solid phase.

13. The method of any one of claims 1 to 12, further comprising prior to step (c) a step of equilibrating the anion exchange solid phase to a pH at which the surface of said solid phase is positively charged using an equilibration buffer.

14. The method of any one of claims 1 to 13, further comprising after step (c) and prior to step (d) one or more step(s) of:
washing the anion exchange solid phase with water or with a washing buffer whereby unbound components are washed away while the nucleic acid(s) remain(s) bound to said solid phase.

15. The method of any one of claims 1 to 14, wherein the target nucleic acid is concentrated concurrently with being purified.
